# EUROPEAN PATENT SPECIFICATION

(11) **EP 4 013 379 B1**
(45) Date of publication and mention of the grant of the patent: **08.01.2025**
(21) Application number: 20740657.0
(22) Date of filing: 22.07.2020
(51) Int. Cl.: A61K 8/92, A61K 8/19, A61K 8/26, A61K 8/34, A61Q 15/00

(54) **NO STAINING ANTIPERSPIRANT COMPOSITION**
NICHT FÄRBENDE ANTITRANSPIRANT-ZUSAMMENSETZUNG
COMPOSITION ANTITRANSPIRATION SANS TRACES

(30) Priority: 12.08.2019 EP 19191290
(43) Date of publication of application: 22.06.2022
(73) Proprietor: Unilever IP Holdings B.V., 3013 AL Rotterdam (NL); Unilever Global IP Limited, Wirral, Merseyside CH62 4ZD (GB)
(72) Inventor: KUNJUPILLAI, Balu, Bangalore 560 066 (IN); NYALAM, Praveen, Bangalore 560 066 (IN); VAIDYA, Ashish, Anant, Bangalore 560 066 (IN)
(74) Representative: Whaley, Christopher
(86) International application number: PCT/EP2020/070730
(87) International publication number: WO 2021/028180

(56) References cited:
- WO-A1-2011/050044
- US-A1- 2003 113 282
- DATABASE GNPD [online] MINTEL; 8 May 2018 (2018-05-08), ANONYMOUS: "48H Anti-Perspirant Deodorant Spray", XP055653733, retrieved from https://www.gnpd.com/sinatra/recordpage/5659805/ Database accession no. 5659805
- DATABASE GNPD [online] MINTEL; 10 September 2018 (2018-09-10), ANONYMOUS: "+Anti Stain 48h Anti-Perspirant Deodorant Roll-On", XP055653726, retrieved from https://www.gnpd.com/sinatra/recordpage/5950573/ Database accession no. 5950573
- DATABASE GNPD [online] MINTEL; 10 September 2018 (2018-09-10), ANONYMOUS: "+Anti Stain 48h Anti-Perspirant Deodorant Roll-On", XP055653726, retrieved from www.gnpd.com Database accession no. 5950573
- DATABASE GNPD [online] MINTEL; 8 May 2018 (2018-05-08), ANONYMOUS: "48H Anti-Perspirant Deodorant Spray", XP055653733, retrieved from www.gnpd.com Database accession no. 5659805

## Description

### Field of the invention

The present invention relates to an antiperspirant (AP) composition. The invention more particularly relates to an antiperspirant composition that comprises conventional metal based AP actives, natural oil and selective phenolic anti-oxidants that ensures that when such compositions are used on a body part e.g. the axilla there is minimal or no yellow coloured staining of the fabric on repeated use of such fabric through several use-wash-rinse-dry cycles.

### Background of the invention

The present invention relates to compositions, such as those that contain antiperspirant actives. These actives are added to compositions to reduce perspiration on application to the surface of the body, particularly to the underarm regions of the human body viz. the axilla. Antiperspirant actives used so far are typically astringent metal salts such as aluminium or zirconium salts e.g. aluminium chlorohydrates or sesquichlorohydrates. Antiperspirant actives are usually incorporated in compositions at low pH, in the range of 2 to 7. Such compositions also include natural oils which may be unsaturated e.g. sunflower seed oil for delivering skin care benefits. Since these oils are unsaturated, antioxidants are generally added to such compositions to minimize oxidation of such compounds to ensure minimal colour formation. Typically, antioxidants like ascorbic acid, butyl hydroxy toluene and other commercially available compounds like Tinogard TT (Pentaerythrityl tetra-di-t-butyl hydroxyhydrocinnamate) or Tinogard Q (Tris - (Tetramethylhydroxy-piperidinol) citrate), both from Ciba, have been used. The present inventors have found that when such conventional antioxidants are used, the fabric which comes in contact with the composition in the axilla tends to get stained on repeated use (where the fabric may be washed, rinsed and dried between each use).

The present inventors have found that when AP compositions comprise very specific phenolic compound preferably tertiary butyl phenolic compounds, it tends to minimize this problem.

US7238343 (Unilever, 2007) discloses antiperspirant composition containing an antiperspirant active, a natural oil and glycerol (in specific weight ratios of natural oil and glycerol) to ameliorate the deleterious effects of hair removal e.g. shaving. WO2011050044 is directed to an antiperspirant composition containing an antioxidant that reduces or eliminates the yellowing on clothing. The yellowing is most noticeable on lighter colored clothing, such as white T-shirts.

There is no prior art where the problem of staining of fabrics on use of antiperspirant composition comprising metal based antiperspirant actives and natural oil has been solved using the specific phenolic antioxidants claimed herein.

It is thus an object of the present invention to provide for an antiperspirant composition comprising a metal based antiperspirant active and natural oil that minimizes the problem of staining or yellowing of fabrics on repeated use-wash-rinse-dry cycles of the fabric when worn by individuals using such antiperspirant compositions.

### Summary of the invention

According to the first aspect of the present invention there is provided an antiperspirant composition comprising
(a) A metal based antiperspirant active;
(b) A natural oil that comprises a glyceride of an unsaturated carboxylic/fatty acid; and
(c) A compound of formula 1 having the structure: 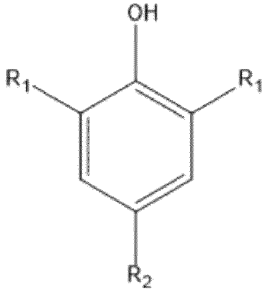 Where
   R₁ is CH₃ or CHR₃R₄ or CR₃R₄R₅ where R₃,R₄,R₅ each independently represents H or linear C₁-C₆ alkyl group or branched C₃-C₆ alkyl group or C₅ - C₆ cyclo alkyl group or a 5 or 6 membered aromatic or heterocyclic group;
   R₂ is H or CHR₃R₄ or CR₃R₄R₅ or XR₃ where X is O or S and where R₃, R₄, R₅ each independently represents linear C₁-C₆ alkyl group or branched C₃-C₆ alkyl group or C₅ - C₆ cyclo alkyl group or a 5 or 6 membered aromatic or heterocyclic group;
   wherein the compound of formula 1 is selected from 2,6-dimethyl-4-(tert-pentyl) phenol; 4-(tert-butyl)-2,6-diethylphenol; 4-isopropyl-2,6-dimethylphenol; 4-(2,3- dimethylbutan-2-yl)-2,6-dimethylphenol; 2,6-diethyl-4-isopropylphenol; 2-(tert-butyl)- 3,6-dimethylphenol; 4-(1-hydroxy-2-methylpropan-2-yl)-2,6-dimethylphenol; 2-(tert- butyl)-4,6-diisopropylphenol; 4-(tert-butyl)-2,6-diisopropylphenol; 2,6-di-tert-butyl-4- methoxyphenol; 4-(sec-butyl)-2,6-di-tert-butylphenol; 2,6-di-tert-butylphenol; 2,4,6-tri- tert-butylphenol; or 4,4'-(propane-2,2-diyl)bis(2,6-dimethylphenol).

According to another aspect of the present invention there is provided a method of minimizing staining or yellowish colouration of fabric comprising the steps of (a) applying a composition of the invention on to a body part preferably the axilla of an individual followed by (b) washing, (c) rinsing and (d) drying the fabric.

According to yet another aspect is provided use of a compound of formula 1 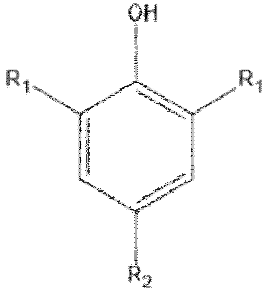 Where
R₁ is CH₃ or CHR₃R₄ or CR₃R₄R₅ where R₃,R₄,R₅ each independently represents H or linear C₁-C₆ alkyl group or branched C₃-C₆ alkyl group or C₅ - C₆ cyclo-alkyl group or a 5 or 6 membered aromatic or heterocyclic group;
R₂ is H or CHR₃R₄ or CR₃R₄R₅ or XR₃ where X is O or S and where R₃, R₄, R₅ each independently represents linear C₁-C₆ alkyl group or branched C₃-C₆ alkyl group or C₅ - C₆ cyclo alkyl group or a 5 or 6 membered aromatic or heterocyclic group ;
wherein the compound of formula 1 is selected from 2,6-dimethyl-4-(tert-pentyl) phenol; 4-(tert-butyl)-2,6-diethylphenol; 4-isopropyl-2,6-dimethylphenol; 4-(2,3- dimethylbutan-2-yl)-2,6-dimethylphenol; 2,6-diethyl-4-isopropylphenol; 2-(tert-butyl)- 3,6-dimethylphenol; 4-(1-hydroxy-2-methylpropan-2-yl)-2,6-dimethylphenol; 2-(tert- butyl)-4,6-diisopropylphenol; 4-(tert-butyl)-2,6-diisopropylphenol; 2,6-di-tert-butyl-4- methoxyphenol; 4-(sec-butyl)-2,6-di-tert-butylphenol; 2,6-di-tert-butylphenol; 2,4,6-tri- tert-butylphenol; or 4,4'-(propane-2,2-diyl)bis(2,6-dimethylphenol)
for minimizing staining of fabrics which are worn by individuals who use the antiperspirant composition according to the first aspect of the invention.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The compositions of the invention are typically "personal care compositions", suitable for use as detailed below. Further, use of the compositions of the invention is typically for cosmetic or non-therapeutic use.

In some embodiments of the present invention, the compositions may be used for the therapeutic treatment of hyperhidrosis (extreme sweating).

By "An Antiperspirant Composition" as used herein, is meant to include a composition for topical application to the skin of mammals, especially humans. Such a composition is preferably of the leave-on type. By a leave-on composition is meant a composition that is applied to the desired skin surface and left on for a period of time (say from one minute to 24 hours) after which it may be wiped or rinsed off with water, usually during the regular course of personal washing. The composition may also be formulated into a product which is applied to a human body for improving the appearance, cleansing, odor control or general aesthetics. The composition of the present invention can be in the form of a liquid, lotion, cream, foam, scrub, gel or stick form. Alternatively they may be delivered through a roll-on device or by using a propellant containing aerosol can. It is especially useful for delivering low pH compositions to the axilla of an individual for anti-perspirancy benefits. "Skin" as used herein is meant to include skin on any part of the body (e.g., neck, chest, back, arms, underarms, hands, legs, buttocks and scalp) especially the underarms.

The anti-perspirant composition as per this invention includes a metal based antiperspirant active. This may be selected from an aluminium, zirconium or mixed aluminium/zirconium salts, preferably, aluminium chlorohydrate, aluminum-zirconium tetrachlorohydrex glycine complex, aluminum-zirconium octachlorohydrex glycine complex, aluminum-zirconium pentachlorohydrate, aluminum sesquichlorohydrate or mixtures thereof.

Antiperspirant actives for use herein are selected from aluminium, zirconium and mixed aluminium/zirconium salts, including both inorganic salts, salts with organic anions and complexes. Particularly preferred astringent salts are halohydrate salts, and especially chlorohydrate salts, optionally activated. For aerosol compositions, the antiperspirant active is preferably free from zirconium.

Aluminium halohydrates are usually defined by the general formula Al₂(OH)ₓQ_{y}.wH₂O in which Q represents chlorine, bromine or iodine, x is variable from 2 to 5 and x + y = 6 while wH₂O represents a variable amount of hydration. Especially effective aluminium halohydrate salts, known as activated aluminium chlorohydrates, are described in EP-A-6739 (Unilever NV et al).

The term aluminium chlorohydrate herein encompasses materials with specified figures for x and y, such as aluminium sesquichlorohydrate and materials in which the chlorohydrate is present as a complex. It will be recognised that alternative names are sometimes used to indicate the presence of hydroxyl substitution, including aluminium hydroxychloride, aluminium oxychloride or basic aluminium chloride.

Zirconium astringent salts for employment herein can usually be represented by the empirical general formula: ZrO(OH)_{2n-nz}B_{z}.wH₂O in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂O. Preferably, B represents chloride. Preferably, the variable z lies in the range from 1.5 to 1.87. In practice, such zirconium salts are commonly not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH. Certain of those Al/Zr complexes are commonly called ZAG in the literature. ZAG actives generally contain aluminium, zirconium and chloride with an Al/Zr ratio in a range from 2 to 10, especially 2 to 6, an AI/CI ratio from 2.1 to 0.9 and a variable amount of glycine. Actives of this preferred type are available from Westwood, from Summit and from Reheis. Alternatively, the complex can be preformed with a polyhydric aliphatic alcohol, such as propylene glycol or glycerol. A complex with a chlorohydrate is commonly referred to as a chlorhydrex.

Mixtures of two or more astringent salts can be employed, but, however, it is particularly preferred to employ astringent salts that are free from zirconium, such as aluminium chlorohydrates and so-called activated aluminium chlorohydrates.

As per an especially preferred aspect of the present invention the antiperspirant active is aluminium chlorohydrate, aluminum sesquichlorohydrate or mixtures thereof. The metal based antiperspirant active preferably is include in 1 to 50%, more preferably 2 to 25%, most preferably 2 to 15% by weight of the composition.

The antiperspirant composition comprises a natural oil. The natural oil is preferably selected from at least one of coriander seed oil, borage seed oil, evening primrose oil, maize corn oil, sunflower oil, safflower oil, coconut oil, algal oil or mixtures thereof. More preferred natural oils are sunflower oil, algal oil or coconut oil preferably sunflower oil. The natural oil is preferably included in 1 to 10%, more preferably 1 to 5% by weight of the composition.

The antiperspirant composition includes a antioxidant which is a phenolic compound having a very specific structure of compound of formula 1:

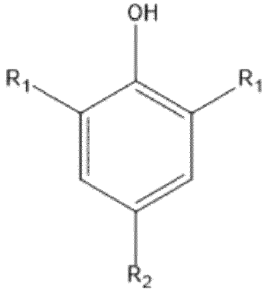

In the above phenolic compound of formula 1 it is preferred that
R₁ is CH₃ or CHR₃R₄ or CR₃R₄R₅ where R₃,R₄,R₅ each independently represents H or linear C₁-C₆ alkyl group or branched C₃-C₆ alkyl group or C₅ - C₆ cyclo alkyl group;
R₂ is H or CHR₃R₄ or CR₃R₄R₅ or XR₃ where X is O or S and where R₃, R₄, R₅ each independently represents linear C₁-C₆ alkyl group or branched C₃-C₆ alkyl group or C₅ - C₆ cyclo alkyl group or a 5 or 6 membered aromatic group, as defined in present claim 1.

The compounds of formula 1 for use in the composition of the present invention are: 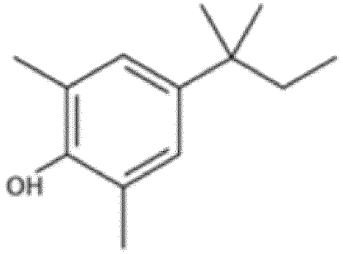 2,6-dimethyl-4-(tert-pentyl) phenol 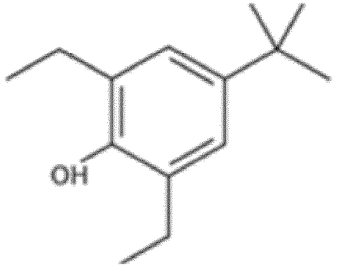 4-(tert-butyl)-2,6-diethylphenol 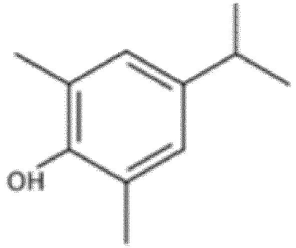 4-isopropyl-2,6-dimethylphenol 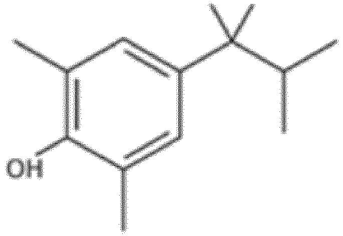 4-(2,3-dimethylbutan-2-yl)-2,6-dimethylphenol 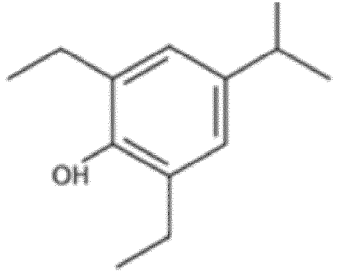 2,6-diethyl-4-isopropylphenol 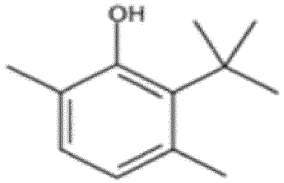 2-(tert-butyl)-3,6-dimethylphenol 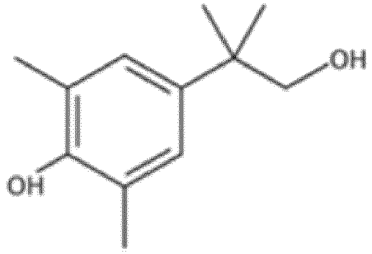 4-(1-hydroxy-2-methylpropan-2-yl)-2,6-dimethylphenol 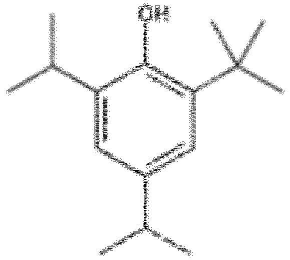 2-(tert-butyl)-4,6-diisopropylphenol 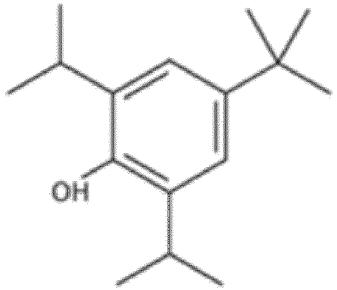 4-(tert-butyl)-2,6-diisopropylphenol 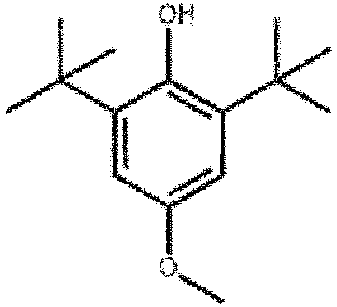 2,6-di-tert-butyl-4-methoxyphenol 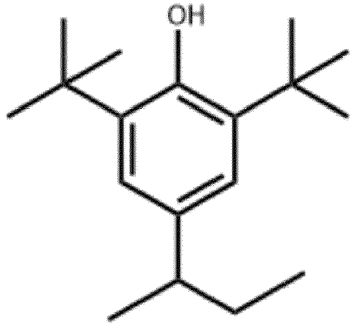 4-(sec-butyl)-2,6-di-tert-butylphenol 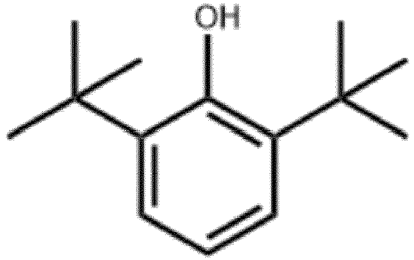 2,6-di-tert-butylphenol 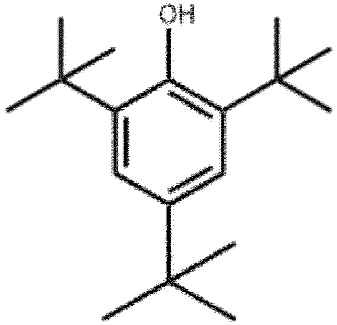 2,4,6-tri-tert-butylphenol 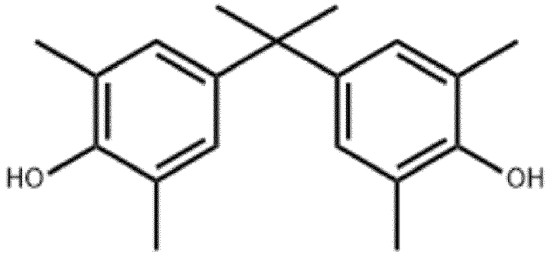 4,4'-(propane-2,2-diyl)bis(2,6-dimethylphenol)

The most preferred compounds of formula 1 for use in the composition of the invention are selected from one or more of 2,6-di-tert-butyl-4-methoxyphenol, 4-(sec-butyl)-2,6-di-tert-butylphenol, 2,6-di-tert-butylphenol, 2,4,6-tri-tert-butylphenol, or 4,4'-(propane-2,2-diyl)bis(2,6-dimethylphenol).

The antiperspirant composition preferably comprises 0.001 to 10%, more preferably 0.01 to 5%, and most preferably 0.05 to 1% the antioxidant compound of formula 1 by weight of the composition. It is particularly preferred that the composition comprises less than 0.05% by weight of an antioxidant other than said compound of formula 1. Ideally, such antioxidant compound other than the compound of formula 1 is absent from the composition.

Without wishing to be bound by theory, the inventors believe that these compounds form stable radical intermediate during their antioxidant activity and reverse back to its original form which stops them from undergoing further oxidation to form chromophore responsible for yellowing

The composition of the invention is preferably delivered as a liquid, lotion, cream, foam, scrub, gel or stick form. Alternately it may be delivered through a roll-on device or using a propellant containing aerosol can. Most preferably the composition is delivered in stick form, through a roll on device or by an aerosol can.

The composition of the invention comprises a topically acceptable carrier. The composition may be delivered when the topically acceptable carrier is anhydrous. By an anhydrous carrier is meant that water content in the composition is less than 5wt%, preferably less than 2 wt%, more preferably less than 1 wt% and optimally absent from the composition. To enable this, the anhydrous carrier preferably comprises a silicone compound, an alcohol or a wax. The alcohol, when used, could be a low boiling (C2-C4) alcohol or a polyhydric alcohol, preferably a polyhydric alcohol.

The pH of the composition is preferably higher than 3.5 more preferably in the range of 4 to 7. The pH of the composition of the invention is measured using the following procedure:
Equal volumes of the composition and model ionic sweat (pH 6.1) are mixed, and the pH value is measured using an accurate range pH test paper.

The composition of the invention preferably comprises a polyhydric alcohol. Polyhydric alcohol is also referred to in short as polyol. A polyhydric alcohol as per the present invention is a compound having two or more hydroxyl groups. Suitable class of polyhydric alcohols that may be included in the composition of the invention are monomeric polyols, polyalkylene glycols or sugars. Preferred monomeric polyols are glycol; alkylene glycol e.g. propylene glycol; glycerol; or xylitol, more preferably propylene glycol.

Suitable polyalkylene glycols are polyethylene glycol or polypropylene glycol. Sugars for inclusion in the invention could be monomeric, dimeric, trimeric or of the polymeric form.

Preferred sugars include glucose, fructose, mannose, sucrose, threitol, erythritol, sorbitol, mannitol, galactitol, adonitol, dextran, or cyclodextrin. Of these the more preferred sugars are glucose, fructose, sucrose, sorbitol, mannitol, adonitol, dextran, or cyclodextrin.

Other components commonly included in conventional antiperspirant compositions may also be incorporated in the composition of the present invention. Such components include skin care agents such as emollients, humectants and skin barrier promoters; skin appearance modifiers such as skin lightening agents and skin smoothing agents; anti-microbial agents, in particular organic anti-microbial agents, and preservatives.

The antiperspirant composition can be applied cosmetically and topically to the skin, broadly speaking, by one of two methods. Some consumers prefer one method and some others, the other method. In one method, sometimes called a contact method, a composition is wiped across the surface of the skin, depositing a fraction of the composition as it passes. In the second method, sometimes called the non-contact method, the composition is sprayed from a dispenser held proximate to the skin, often in an area of about 10 to 20 cm². The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

### Stick or soft solid compositions

Many different materials have been proposed as gellant for a continuous oil phase, including waxes, small molecule gelling agents and polymers. They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

### Roll-on

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

### Aerosol compositions

The antiperspirant composition may be delivered through an aerosol composition which comprises a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; i) low boiling point gasses liquifided by compression, ii) volatile ethers and iii) compressed non-oxidising gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative.

When the composition of the invention is delivered in a roll-on, a firm solid or a stick format, the topically acceptable carrier comprises a hydrophobic carrier or an aqueous carrier. The hydrophobic carrier in such cases may comprise a silicone compound, low boiling alcohol or a wax. When the composition comprises a propellant it is delivered as an aerosol.

The composition of the present invention can comprise a wide range of other optional components. The CTFA Personal care Ingredient Handbook, Second Edition, 1992, describes a wide variety of non-limiting personal care and pharmaceutical ingredients commonly used in the skin care industry, which are suitable for use in the compositions of the present invention. Examples include: binders, biological additives, buffering agents, colorants, thickeners, polymers, astringents, fragrance, conditioners, exfoliating agents, pH adjusters, preservatives, natural extracts, essential oils, skin sensates, skin soothing agents, and skin healing agents.

According to another aspect of the present invention there is provided a method of minimizing staining or yellowish colouration of fabric comprising the steps of (a) applying a composition of the invention preferably the axilla of an individual followed by (b) washing, (c) rinsing and (d) drying the fabric.

The composition is preferably applied on the axilla. The method is also preferably non-therapeutic or for cosmetic application.

Yet another aspect of the present invention relates to use of compound of formula 1 for manufacture of a composition of the invention, for minimizing staining of fabrics which are worn by individuals who use the composition.

The invention will now be illustrated with the help of the following non-limiting examples.

### Examples

Compositions as shown in Table 1 below were prepared:

**Table -1**

| Ingredient | wt% |
|---|---|
| Aluminium chlorohydrate | 12.0 |
| Steareth-20 | 0.9 |
| Steareth-2 | 2.3 |
| Silica dimethyl silylate | 0.7 |
| Sunflower seed oil | 2.0 |
| Antioxidant | As in Table - 2 |
| Antioxidant (wt%) | 0.05 |
| Minors and Water | To 100 |

**Table - 2**

| Example - 1 | Antioxidant |
|---|---|
| A | 4,4-Methylenebis(2,6-di-tert-butylphenol) |
| B | Octadecyl 3-(3,5-di-tert-butyl-4-hydroxyphenyl)propionate |
| C | Pentaerythrityl Tetra-di-t-butyl Hydroxyhydrocinnamate |
| 1 | 2,4,6-tert butyl phenol |
| 2 | 4,4-Isopropylidenebis(2,6-dimethylphenol) |
| 3 | 2,6-di-tert-Buty phenol |
| 4 | 4-sec-Butyl-2,6-di-tert-butylphenol |
| 5 | 2,6-Di-tert-butyl-4-methoxyphenol |

The above compositions were evaluated for staining of fabric using the following protocol

### Please find the protocol used for the evaluation

750 mg of Antiperspirant composition was applied on 100 % cotton fabric swatches(n=5) (WFK 10 A, supplier- Center for Testmaterials) of 33 cm² (Diameter: 6.5 cm) area. The fabric swatches were incubated at 45° C and 80 % humidity for 12 hrs. in a humidity chamber. The fabric swatches were removed from the incubator and washed in Tergotometer with powder detergent and dried in dark room for 10 days. The stain intensity as L* a* b* was measured using spectrophotometer at three different stain area and Δ E was calculated compared to control fabric. Δ E is indicative of total color change across the whole visible spectrum. The lower the value, the lesser the stain.

Each of the above compositions were treated using the above procedure and the intensity of the stain was measured and the average of 15 readings (3 * 5 swatches) is summarised in Table - 3 below:)

**Table - 3:**

| Example | Δ E values |
|---|---|
| A | 16.4 |
| B | 7.3 |
| C | 2.0 |
| 1 | 1.1 |
| 2 | 1.4 |
| 3 | 1.1 |
| 4 | 1.3 |
| 5 | 1.0 |

The data in Table - 3 above indicates that composition as per the invention (Examples - 1 to 5) gives much lower stain intensity and statistically significant (P<0.0001) as compared to using compositions outside the invention (Example A to C).

## Claims

1. An antiperspirant composition comprising
(a) A metal based antiperspirant active;
(b) A natural oil that comprises a glyceride of an unsaturated carboxylic/fatty acid; and
(c) A compound of formula 1 having the structure: 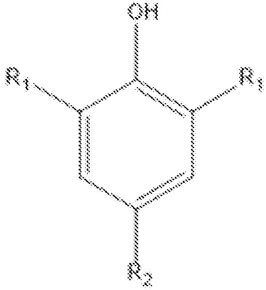 Where
R₁ is CH₃ or CHR₃R₄ or CR₃R₄R₅ where R₃,R₄,R₅ each independently represents H or linear C₁-C₆ alkyl group or branched C₃-C₆ alkyl group or C₅ - C₆ cyclo alkyl group or a 5 or 6 membered aromatic or heterocyclic group;
R₂ is H or CHR₃R₄ or CR₃R₄R₅ or XR₃ where X is O or S and where R₃,R₄, R₅ each independently represents linear C₁-C₆ alkyl group or branched C₃-C₆ alkyl group or C₅ - C₆ cyclo alkyl group or a 5 or 6 membered aromatic or heterocyclic group;
wherein the compound of formula 1 is selected from 2,6-dimethyl-4-(tert-pentyl) phenol; 4-(tert-butyl)-2,6-diethylphenol; 4-isopropyl-2,6-dimethylphenol; 4-(2,3-dimethylbutan-2-yl)-2,6-dimethylphenol; 2,6-diethyl-4-isopropylphenol; 2-(tert-butyl)-3,6-dimethylphenol; 4-(1-hydroxy-2-methylpropan-2-yl)-2,6-dimethylphenol; 2-(tert-butyl)-4,6-diisopropylphenol; 4-(tert-butyl)-2,6-diisopropylphenol; 2,6-di-tert-butyl-4-methoxyphenol; 4-(sec-butyl)-2,6-di-tert-butylphenol; 2,6-di-tert-butylphenol; 2,4,6-tri-tert-butylphenol; or 4,4'-(propane-2,2-diyl)bis(2,6-dimethylphenol).

2. An antiperspirant composition as claimed in claim 1 wherein said metal based antiperspirant active is selected from an aluminium, zirconium or mixed aluminium/zirconium salt, preferably, aluminium chlorohydrate, aluminum-zirconium tetrachlorohydrex glycine complex, aluminum-zirconium octachlorohydrex glycine complex, aluminum-zirconium pentachlorohydrate, aluminum sesquichlorohydrate or mixtures thereof.

3. An antiperspirant composition as claimed in claim 2 wherein said metal based antiperspirant active is aluminium chlorohydrate, aluminum sesquichlorohydrate or mixtures thereof.

4. An antiperspirant composition as claimed in any one of the preceding claims comprising 1 to 50% metal based antiperspirant active by weight of the composition.

5. An antiperspirant composition as claimed in claim any one of the preceding claims wherein said natural oil is selected from at least one of coriander seed oil, borage seed oil, evening primrose oil, maize corn oil, sunflower oil, safflower oil, coconut oil, and algal oil.

6. An antiperspirant composition as claimed in claim 5 wherein said natural oil is sunflower oil, algal oil or coconut oil preferably sunflower oil.

7. An antiperspirant composition as claimed in any one of the preceding claims comprising 1 to 10% natural oil by weight of the composition.

8. An antiperspirant composition as claimed in any one of the preceding claims wherein the compound of formula 1 is selected from one or more of 2,6-di-tert-butyl-4-methoxyphenol, 4-(sec-butyl)-2,6-di-tert-butylphenol, 2,6-di-tert-butylphenol, 2,4,6-tri-tert-butylphenol, or 4,4'-(propane-2,2-diyl)bis(2,6-dimethylphenol).

9. An antiperspirant composition as claimed in any one of the preceding claims comprising 0.001 to 10% compound of formula 1 by weight of the composition.

10. An antiperspirant composition as claimed in any one of the preceding claims comprising less than 0.05% by weight of an antioxidant other than said compound of formula 1.

11. An antiperspirant composition as claimed in any one of the preceding claims which is delivered in a stick form, from a roll on device or in an aerosol can.

12. A method of minimizing staining or yellowish colouration of fabric comprising the steps of (a) applying a composition as claimed in any one of the preceding claims on to a body part preferably the axilla of an individual followed by (b) washing, (c) rinsing and (d) drying the fabric.

13. Use of a compound of formula 1 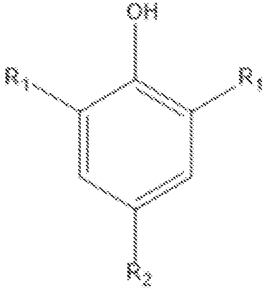 Where
R₁ is CH₃ or CHR₃R₄ or CR₃R₄R₅ where R₃,R₄,R₅ each independently represents H or linear C₁-C₆ alkyl group or branched C₃-C₆ alkyl group or C₅ - C₆ cyclo-alkyl group or a 5 or 6 membered aromatic or heterocyclic group;
R₂ is H or CHR₃R₄ or CR₃R₄R₅ or XR₃ where X is O or S and where R₃,R₄, R₅ each independently represents linear C₁-C₆ alkyl group or branched C₃-C₆ alkyl group or C₅ - C₆ cyclo-alkyl group or a 5 or 6 membered aromatic or heterocyclic group,
wherein the compound of formula 1 is selected from 2,6-dimethyl-4-(tert-pentyl) phenol; 4-(tert-butyl)-2,6-diethylphenol; 4-isopropyl-2,6-dimethylphenol; 4-(2,3-dimethylbutan-2-yl)-2,6-dimethylphenol; 2,6-diethyl-4-isopropylphenol; 2-(tert-butyl)-3,6-dimethylphenol; 4-(1-hydroxy-2-methylpropan-2-yl)-2,6-dimethylphenol; 2-(tert-butyl)-4,6-diisopropylphenol; 4-(tert-butyl)-2,6-diisopropylphenol; 2,6-di-tert-butyl-4-methoxyphenol; 4-(sec-butyl)-2,6-di-tert-butylphenol; 2,6-di-tert-butylphenol; 2,4,6-tri-tert-butylphenol; or 4,4'-(propane-2,2-diyl)bis(2,6-dimethylphenol) for minimizing staining of fabrics which are worn by individuals who use the composition according to any of claims 1 to 13.

## Patentansprüche

1. Eine Antitranspirant-Zusammensetzung, umfassend
(a) einen Antitranspirant-Wirkstoff auf Metallbasis;
(b) ein natürliches Öl, das ein Glycerid einer ungesättigten Carbon-/Fettsäure umfasst; und
(c) eine Verbindung der Formel 1 mit der Struktur: 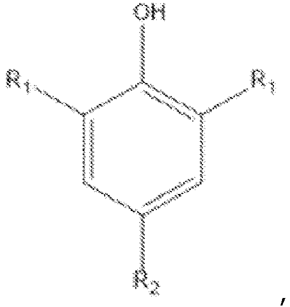 wobei
R₁ CH₃ oder CHR₃R₄ oder CR₃R₄R₅ ist, wobei R₃, R₄, R₅ jeweils unabhängig voneinander H oder eine lineare C₁-C₆-Alkylgruppe oder eine verzweigte C₃-C₆-Alkylgruppe oder eine C₅-C₆-Cycloalkylgruppe oder eine 5- oder 6-gliedrige aromatische oder heterocyclische Gruppe darstellen;
R₂ H oder CHR₃R₄ oder CR₃R₄R₅ oder XR₃ ist, wobei X O oder S ist und wobei R₃, R₄, R₅ jeweils unabhängig voneinander eine lineare C₁-C₆-Alkylgruppe oder eine verzweigte C₃-C₆-Alkylgruppe oder eine C₅-C₆-Cycloalkylgruppe oder eine 5- oder 6-gliedrige aromatische oder heterocyclische Gruppe darstellen;
wobei die Verbindung der Formel 1 ausgewählt ist unter 2,6-Dimethyl-4-(tert-pentyl)phenol, 4-(tert-Butyl)-2,6-diethylphenol, 4-Isopropyl-2,6-dimethylphenol, 4-(2,3-Dimethylbutan-2-yl)2,6-dimethylphenol, 2,6-Diethyl-4-isopropylphenol, 2-(tert-Butyl)-3,6-dimethylphenol, 4-(1-Hydroxy-2-methylpropan-2-yl)-2,6-dimethylphenol, 2-(tert-Butyl)-4,6-diisopropylphenol, 4-(tert-Butyl)-2,6-diisopropylphenol, 2,6-Di-tert-butyl-4-methoxyphenol, 4-(sec-Butyl)-2,6-di-tert-butylphenol, 2,6-Di-tert-butylphenol, 2,4,6-Tri-tert-butylphenol oder 4,4'-(Propan-2,2-diyl)bis(2,6-dimethylphenol).

2. Antitranspirant-Zusammensetzung, wie im Anspruch 1 beansprucht, wobei der Antitranspirant-Wirkstoff auf metallischer Basis unter einem Aluminium-, Zirkonium- oder gemischten Aluminium-/Zirkoniumsalz, bevorzugt einem Aluminiumchlorhydrat, einem Aluminium-Zirkonium-tetrachlorhydrex-Glycin-Komplex, einem Aluminium-Zirkonium-octachlorhydrex-Glycin-Komplex, einem Aluminium-Zirkonium-pentachlorhydrat, einem Aluminium-sesquichlorhydrat oder Mischungen davon ausgewählt ist.

3. Antitranspirant-Zusammensetzung, wie im Anspruch 2 beansprucht, wobei der Antitranspirant-Wirkstoff auf metallischer Basis Aluminiumchlorhydrat, Aluminiumsesquichlorhydrat oder Mischungen davon darstellt.

4. Antitranspirant-Zusammensetzung, wie in irgendeinem der vorhergehenden Ansprüche beansprucht, umfassend, bezogen auf das Gewicht der Zusammensetzung, 1 bis 50% Antitranspirant-Wirkstoff auf metallischer Basis.

5. Antitranspirant-Zusammensetzung, wie in irgendeinem der vorhergehenden Ansprüche beansprucht, wobei das natürliche Öl unter mindestens einem von Koriandersamenöl, Borretschsamenöl, Nachtkerzenöl, Maisöl, Sonnenblumenöl, Safloröl, Kokosnussöl und Algenöl ausgewählt ist.

6. Antitranspirant-Zusammensetzung, wie im Anspruch 5 beansprucht, wobei das natürliche Öl Sonnenblumenöl, Algenöl oder Kokosnussöl, vorzugsweise Sonnenblumenöl, ist.

7. Antitranspirant-Zusammensetzung, wie in irgendeinem der vorhergehenden Ansprüche beansprucht, umfassend, bezogen auf das Gewicht der Zusammensetzung, 1 bis 10% natürliches Öl.

8. Antitranspirant-Zusammensetzung, wie in irgendeinem der vorhergehenden Ansprüche beansprucht, wobei die Verbindung der Formel 1 unter ein oder mehreren Verbindungen von 2,6-Di-tert-butyl-4-methoxyphenol, 4-(sec-Butyl)-2,6-di-tert-butylphenol, 2,6-Di-tert-butylphenol, 2,4,6-Tri-tert-butylphenol oder 4,4'-Propan-2,2-diyl)bis(2,6-dimethylphenol) ausgewählt ist.

9. Antitranspirant-Zusammensetzung, wie in irgendeinem der vorhergehenden Ansprüche beansprucht, umfassend, bezogen auf das Gewicht der Zusammensetzung, 0,001 bis 10% Verbindung der Formel 1.

10. Antitranspirant-Zusammensetzung, wie in irgendeinem der vorhergehenden Ansprüche beansprucht, umfassend weniger als 0,05 Gewichts-% eines sich von der Verbindung der Formel 1 unterscheidenden Antioxidationsmittels.

11. Antitranspirant-Zusammensetzung, wie in irgendeinem der vorhergehenden Ansprüche beansprucht, die in Stiftform, von einem Roll-on-Gerät oder in einer Aerosoldose geliefert wird.

12. Verfahren zum Minimieren der Fleckenbildung oder der Gelbfärbung von Textilien, umfassend die Schritte (a) Auftragen einer Zusammensetzung, wie irgendeinem der vorhergehenden Ansprüche beansprucht, auf ein Körperteil, vorzugsweise auf die Achselhöhle einer Person, gefolgt von (b) Waschen, (c) Spülen und (d) Trocknen des Textils.

13. Verwendung einer Verbindung der Formel 1 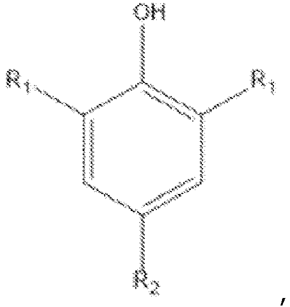 wobei
R₁ CH₃ oder CHR₃R₄ oder CR₃R₄R₅ ist, wobei R₃, R₄, R₅ jeweils unabhängig voneinander H oder eine lineare C₁-C₆-Alkylgruppe oder eine verzweigte C₃-C₆-Alkylgruppe oder eine C₅-C₆-Cycloalkylgruppe oder eine 5- oder 6-gliedrige aromatische oder heterocyclische Gruppe darstellen;
R₂ H oder CHR₃R₄ oder CR₃R₄R₅ oder XR₃ ist, wobei X O oder S ist und wobei R₃, R₄, R₅ jeweils unabhängig voneinander eine lineare C₁-C₆-Alkylgruppe oder eine verzweigte C₃-C₆-Alkylgruppe oder eine C₅-C₆-Cycloalkylgruppe oder eine 5- oder 6-gliedrige aromatische oder heterocyclische Gruppe darstellen;
wobei die Verbindung der Formel 1 ausgewählt ist unter 2,6-Dimethyl-4-(tert-pentyl)phenol, 4-(tert-Butyl)-2,6-diethylphenol, 4-Isopropyl-2,6-dimethylphenol, 4-(2,3-Dimethylbutan-2-yl)2,6-dimethylphenol, 2,6-Diethyl-4-isopropylphenol, 2-(tert-Butyl)-3,6-dimethylphenol, 4-(1-Hydroxy-2-methylpropan-2-yl)-2,6-dimethylphenol, 2-(tert-Butyl)-4,6-diisopropylphenol, 4-(tert-Butyl)-2,6-diisopropylphenol, 2,6-Di-tert-butyl-4-methoxyphenol, 4-(sec-Butyl)-2,6-di-tert-butylphenol, 2,6-Di-tert-butylphenol, 2,4,6-Tri-tert-butylphenol oder 4,4'-(Propan-2,2-diyl)bis(2,6-dimethylphenol)
zur Minimierung der Fleckenbildung auf Textilien, die von Personen getragen werden, die die Zusammensetzung nach einem der Ansprüche 1 bis 13 verwenden.

## Revendications

1. Composition anti-transpirante comprenant
(a) un actif anti-transpirant à base de métal ;
(b) une huile naturelle qui comprend un glycéride d'un acide carboxylique/gras insaturé ; et
(c) un composé de formule 1 ayant la structure : 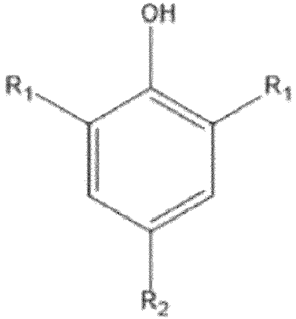 où
R₁ est CH₃ ou CHR₃R₄ ou CR₃R₄R₅ où R₃, R₄, R₅ représentent chacun indépendamment H ou un groupe alkyle en C₁ à C₆ linéaire ou un groupe alkyle en C₃ à C₆ ramifié ou un groupe cycloalkyle en C₅ à C₆ ou un groupe aromatique ou hétérocyclique à 5 ou 6 chaînons ;
R₂ est H ou CHR₃R₄ ou CR₃R₄R₅ ou XR₃ où X est O ou S et où R₃, R₄, R₅ représentent chacun indépendamment un groupe alkyle en C₁ à C₆ linéaire ou un groupe alkyle en C₃ à C₆ ramifié ou un groupe cycloalkyle en C₅ à C₆ ou un groupe aromatique ou hétérocyclique à 5 ou 6 chaînons ;
dans laquelle le composé de formule 1 est choisi parmi le 2,6-diméthyl-4-(tert-pentyl)phénol ; le 4-(tert-butyl)-2,6-diéthylphénol ; le 4-isopropyl-2,6-diméthylphénol ; le 4-(2,3-diméthylbutan-2-yl)-2,6-diméthylphénol ; le 2,6-diéthyl-4-isopropylphénol ; le 2-(tert-butyl)-3,6-diméthylphénol ; le 4-(1-hydroxy-2-méthylpropan-2-yl)-2,6-diméthylphénol ; le 2-(tert-butyl)-4,6-diisopropylphénol ; le 4-(tert-butyl)-2,6-diisopropylphénol ; le 2,6-di-tert-butyl-4-méthoxyphénol ; le 4-(sec-butyl)-2,6-di-tert-butylphénol ; le 2,6-di-tert-butylphénol ; le 2,4,6-tri-tert-butylphénol ; ou le 4,4'-(propane-2,2-diyl)bis(2,6-diméthylphénol).

2. Composition anti-transpirante selon la revendication 1 dans laquelle ledit actif anti-transpirant à base de métal est choisi parmi un sel d'aluminium, de zirconium ou d'aluminium/zirconium mixtes, de préférence le chlorhydrate d'aluminium, le complexe aluminium-zirconium tétrachlorohydrex glycine, le complexe aluminium-zirconium octachlorohydrex glycine, le pentachlorohydrate d'aluminium-zirconium, le sesquichlorohydrate d'aluminium ou des mélanges de ceux-ci.

3. Composition anti-transpirante selon la revendication 2 dans laquelle ledit actif anti-transpirant à base de métal est le chlorhydrate d'aluminium, le sesquichlorohydrate d'aluminium ou des mélanges de ceux-ci.

4. Composition anti-transpirante selon l'une quelconque des revendications précédentes comprenant de 1 à 50 % d'actif anti-transpirant à base de métal en poids de la composition.

5. Composition anti-transpirante selon l'une quelconque des revendications précédentes dans laquelle ladite huile naturelle est choisie parmi au moins l'une parmi l'huile de coriandre, l'huile de bourrache, l'huile d'onagre, l'huile de maïs, l'huile de tournesol, l'huile de carthame, l'huile de coco et l'huile d'algue.

6. Composition anti-transpirante selon la revendication 5 dans laquelle ladite huile naturelle est l'huile de tournesol, l'huile d'algue ou l'huile de coco, de préférence l'huile de tournesol.

7. Composition anti-transpirante selon l'une quelconque des revendications précédentes comprenant de 1 à 10 % d'huile naturelle en poids de la composition.

8. Composition anti-transpirante selon l'une quelconque des revendications précédentes dans laquelle le composé de formule 1 est choisi parmi un ou plusieurs parmi le 2,6-di-tert-butyl-4-méthoxyphénol, le 4-(sec-butyl)-2,6-di-tert-butylphénol, le 2,6-di-tert-butylphénol, le 2,4,6-tri-tert-butylphénol, ou le 4,4'-(propane-2,2-diyl)bis(2,6-diméthylphénol).

9. Composition anti-transpirante selon l'une quelconque des revendications précédentes comprenant de 0,001 à 10 % de composé de formule en poids de la composition.

10. Composition anti-transpirante selon l'une quelconque des revendications précédentes comprenant moins de 0,05 % en poids d'un antioxydant autre que ledit composé de formule 1.

11. Composition anti-transpirante selon l'une quelconque des revendications précédentes qui est délivrée sous forme de stick, à partir d'un dispositif d'application à bille ou dans une bombe aérosol.

12. Procédé de minimisation des taches ou de la coloration jaunâtre d'un tissu comprenant les étapes consistant à (a) appliquer une composition selon l'une quelconque des revendications précédentes sur une partie du corps, de préférence l'aisselle d'une personne, puis (b) laver, (c) rincer et (d) sécher le tissu.

13. Utilisation d'un composé de formule 1 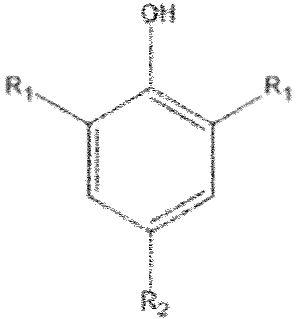 où
R₁ est CH₃ ou CHR₃R₄ ou CR₃R₄R₅ où R₃, R₄, R₅ représentent chacun indépendamment H ou un groupe alkyle en C₁ à C₆ linéaire ou un groupe alkyle en C₃ à C₆ ramifié ou un groupe cycloalkyle en C₅ à C₆ ou un groupe aromatique ou hétérocyclique à 5 ou 6 chaînons ;
R₂ est H ou CHR₃R₄ ou CR₃R₄R₅ ou XR₃ où X est O ou S et où R₃, R₄, R₅ représentent chacun indépendamment un groupe alkyle en C₁ à C₆ linéaire ou un groupe alkyle en C₃ à C₆ ramifié ou un groupe cycloalkyle en C₅ à C₆ ou un groupe aromatique ou hétérocyclique à 5 ou 6 chaînons ;
dans laquelle le composé de formule 1 est choisi parmi le 2,6-diméthyl-4-(tert-pentyl)phénol ; le 4-(tert-butyl)-2,6-diéthylphénol ; le 4-isopropyl-2,6-diméthylphénol ; le 4-(2,3-diméthylbutan-2-yl)-2,6-diméthylphénol ; le 2,6-diéthyl-4-isopropylphénol ; le 2-(tert-butyl)-3,6-diméthylphénol ; le 4-(1-hydroxy-2-méthylpropan-2-yl)-2,6-diméthylphénol ; le 2-(tert-butyl)-4,6-diisopropylphénol ; le 4-(tert-butyl)-2,6-diisopropylphénol ; le 2,6-di-tert-butyl-4-méthoxyphénol ; le 4-(sec-butyl)-2,6-di-tert-butylphénol ; le 2,6-di-tert-butylphénol ; le 2,4,6-tri-tert-butylphénol ; ou le 4,4'-(propane-2,2-diyl)bis(2,6-diméthylphénol)
pour minimiser les taches sur les tissus qui sont portés par les personnes qui utilisent la composition anti-transpirante selon l'une quelconque des revendications 1 à 13.
